# EUROPEAN PATENT APPLICATION

(11) **EP 3 203 350 A1**
(43) Date of publication of application: **09.08.2017**
(21) Application number: 16275066.5
(22) Date of filing: 26.04.2016
(51) Int. Cl.: G06F 1/16, G06F 3/01, G06F 1/32, A61B 5/00

(54) **WEARABLE CONTROLLER FOR WRIST**

(30) Priority: 03.02.2016 US 201615014021
(71) Applicant: FlickTek Ltd, London SW1V 1DW (GB)
(72) Inventor: BELFIORI, Alfredo, Quarta Sant'Elena 09045 (IT)
(74) Representative: Barnes, Philip Michael

(57) **Abstract**

A wrist-worn computer interface including a sensor for measuring wrist tendon forces corresponding to specific finger motions including a linear array of cantilevered piezoelectric sensors configured to emit electric currents upon pressure from the wrist tendons on the tip of the piezoelectric sensors, a processing module configured for converting the electric currents generated upon pressure from wrist tendons into signals and for processing the signals to identify one or more specific finger motions, and a flexible PCB connecting the piezoelectric sensors to the processing module. A controller module is configured to cause one or more computing devices to automatically execute one or more specific commands corresponding to one or more of the specific finger motions.

## Description

### BACKGROUND

### Technical Field

This disclosure relates to the field of the wearable devices, biometric controller and interfaces and biomedical engineering. More specifically this disclosure is directed to a human-computer interface and a system for providing the same. According to an aspect of the disclosure, a controller, placed around the wrist, able to read fingers movements is described. The disclosure is also directed to a process for sending commands to one or more computing devices by means of a wearable device.

### Description of the Related Art

The technology that has been used as an interface between human and machine had so far faced two big revolutions. Initially large electronic companies produced interfaces based on the use of buttons, such as control panels, keyboards and mouse. This technology was later replaced by touchscreen, an innovative solution that does not require physical buttons but is responsive to the contact of a body part such as fingertip. Next step in the human-machine interface evolution will be the elimination of physical contact between the user and the device controlled. This will be achieved using the biometric body signals, such as the electric, mechanical or vibrational phenomena related to muscular contractions, to control any electronic device.

### SUMMARY

This disclosure is directed to a human-computer interface (HCI) able to detect and interpret fingers gestures and send the information to any electronic device, particularly a computing device. More specifically the disclosure teaches a controller consisting of an array of cantilever piezoelectric sensors for detecting movements of tendons in the wrist corresponding to specific finger gestures.

In an embodiment, the disclosure teaches a wrist-worn sensor for measuring wrist tendon forces corresponding to specific finger motions including an array of cantilever piezoelectric sensors where the piezoelectric sensors emit electric currents generated upon pressure from the wrist tendons on the tip of the piezoelectric sensors, a processing module configured for converting the electric currents into signals and processing the signals for identification of one or more specific finger motions, and a flexible PCB connecting the piezoelectric sensors to the processing module.

In an aspect of the disclosure, the array of piezoelectric sensors is configured to have a spatial resolution of less than 2 mm.

In yet another aspect, the cantilever sensors are configured in a linear array.

In still another aspect, the linear array comprises four piezo-electric sensors with partially overlapping sensor areas.

In yet another aspect, the array of cantilever piezoelectric sensors is positioned proximally to a wearer's Flexor Carpi Ulnaris Tendon, Flexor Digitorum Profundus Tendon and Flexor Digitorum Superficialis Tendon.

In another aspect of the disclosure, the array of cantilever piezoelectric sensors is configured to optimally capture the tension applied to each tendon in the wrist.

In an embodiment of the disclosure, the sensors are positioned at an angle greater than 10 degrees relative to the flexible PCB.

In another embodiment, the piezoelectric sensors are embedded in an elastomeric material.

In a preferred embodiment, the elastomeric material is selected from the list consisting of silicone rubber, polymer foam and polymer elastomer.

In an aspect of the disclosure, the elastomeric material filters out low amplitude high frequency signals.

In an embodiment of the human computer interface, a controller module is configured to cause one or more computing devices to automatically execute one or more specific commands upon identification of one or more of the specific finger motions.

In another embodiment, the computer interface communicates wirelessly with one or more computing devices.

In yet another embodiment, the human computer interface includes a button placed in in contact with a user's wrist so as to be triggered by the user flexing the wrist and causing the activation of the device from a sleeping, power-saving mode to an active acquisition mode.

In another embodiment, the wrist-worn sensor includes a controller module configured to cause one or more computing devices to automatically execute one or more specific commands upon identification of one or more of the specific finger motions.

More specifically, a wearable wrist controller is described which includes an array of cantilever piezoelectric sensors in order to monitor the movements of muscles, tendons and other body tissues, the combination of their movements in the wrist and their initial and final positions.

The present disclosure provides a wired or wireless HCI for interacting with computing systems and attached devices via electrical signals generated by specific movement of the user's fingers. The specific movements follow a fixed protocol. Following an initial automated calibration process, measurement and interpretation of signals generated by finger movements is accomplished by sampling signals with the cantilever piezoelectric sensors of the wrist worn wearable controller. In operation, the wrist worn wearable controller is donned by the user and placed into a fixed position on the surface of the user's wrist skin. Automated cues or instructions are then provided to the user for fine-tuning control of the wearable controller for the wrist. Examples of wearable controllers for the wrist include articles of manufacture, such as a wristband, wristwatch, or articles of clothing having a plurality of integrated piezo-electric sensor nodes, and associated electronics.

According to an aspect of the disclosure, a calibration phase is provided for the human-computer interface. The calibration phase automatically identifies the parameters needed to run a software program installed in the module, or in one or more external computing devices. The software receives the signals and identifies the parameter for the training following a protocol of specific finger gestures.

The calibration phase of the human-computer interface which is described herein involves the user performing one or more specific finger gestures as part of a training phase for the calibration phase. This allows to precisely tune the performance of the interface to the specific biometry of the user.

More specifically, the sensors provided in the device measure the signals that are associated with one or more of the specific user gestures.

More specifically, the human-computer interface further comprises a module for automatically determining the position of the signal source on the surface of the skin of the user's wrist, in order to identify which finger moved and how.

According to another aspect of the disclosure, the human-computer interface of the disclosure is used in a process for detecting specific finger movements based on wrist-tendon forces, the process comprising the steps of:
a) sensing one or more electric signals produced by an array of cantilever piezoelectric sensors from the pressure of wrist tendons applied to the tip of the sensors;
b) extracting a set of characteristic features from the electric signal;
c) feeding the characteristic features to a trained classifier;
d) identifying one or more specific finger gestures associated with specific classes of the trained classifier; and
e) automatically directing one or more computing devices to execute one or more commands corresponding to one or more of the identified finger gestures.

In an aspect of the disclosure, the process further includes the step of performing an initial calibration of the sensors which evaluates gesture generated signals associated with a subset of user finger gestures to determine expected signals during the finger-gesture identification step.

In another aspect of the disclosure, the process further includes the step of calibrating the controller by automatically identifying the parameters needed to run a software program installed in the module or in one or more external computing devices, the software program receiving the signals and identifying the parameter for the training following a protocol of specific finger gestures.

In yet another aspect of the disclosure, the feature extraction step further includes the steps of considering all electric signals coming from the sensors during each finger movement and gesture, band-pass filtering said signals to limit the data to a predetermined amount, and analyzing the signals by means of a feature extractor.

In still another aspect of the disclosure, the feature extraction step further analyzes the signals in order to obtain a set of features describing the signals to be compared with other signal features coming from other finger movements and gestures.

In another aspect, the features are selected from the list consisting of time domain features and frequency domain features.

In another aspect of the disclosure, the process further includes the step of a step of disabling one or more of the sensors during rest.

The process involves positioning the wearable device in contact with the surface of a user's wrist skin. Through the one or more piezoelectric cantilever sensors, the state and the activity of the different body tissues at the user's wrist are then measured. The process also involves automatically evaluating gesture-generated signals of the user, which are measured via the one or more of cantilever piezoelectric sensors, in order to automatically identify one or more specific gestures of the user from a predefined set of gestures.

Preferably, the process further comprises performing an initial calibration phase which evaluates gesture generated signals associated with a subset of user finger gestures to determine expected signals during the automatic evaluation phase. According to a preferred feature, commands associated with one or more of the gestures of the set of gestures can be user definable.

According to another aspect of the disclosure, a system for providing a human-computer interface (HCI) comprises a user-wearable device having one or more cantilever piezoelectric sensors. The user-wearable device is configured to be placed against the surface of the user's wrist. The system also comprises an automated calibration process which maps finger gestures generated signals corresponding to one or more specific user finger gestures to one or more specific commands. The finger gestures generated signals are measured by a linear or non-linear array of cantilever piezoelectric sensors. Furthermore, the system comprises an automated process for disabling some of said sensors during rest, and an automated process for evaluating one or more user gestures associated with the signals captured by the sensor array and to identify one or more commands associated with those user gestures. The system also comprises a process for transmitting specific commands associated with one or more specific user gestures to one or more computing devices.

Preferably, the user-wearable device of the above-mentioned system includes a wireless or wired interface to the one or more computing devices.

The two greatest advantages of controlling a device using the controller is that the physical contact such as controlling a smartphone through a touchscreen is no longer required. The finger movement made without visual control, triggers the controller to send a control signal or some data to other electronic devices.

In view of the above summarized capabilities, and in further view of the following detailed description, it should be understood that the controller provides users with a "universal" input mechanism that can be used to control any computing device, applications running of computing devices, electronic or mechanical devices coupled to a computing device, or any other electronic device (television, radio, appliance, light switch, etc.) having an appropriate infrastructure or interface for receiving input from a wired or wireless controller. Note also that the use of a small wearable device such as the controller, which may be under the user's clothes, if desired, provides a mechanism that is unobtrusive (i.e., the user can be using her hands to perform other tasks while using controller to provide active control of one or more devices). Further, it should also be appreciated that the control and interface capabilities provided by the controller are potentially invisible in the sense that a user wearing one or more such controllers can remotely interact with various devices without anyone else being able to see or hear any overt actions by the user to indicate that the user is interacting with such devices.

Some of the advantages offered by the controller are that the controller enables finger movements generated signal to control computing devices, applications, and attached devices with little preparation and setup on the part of the user. In fact, in the simplest embodiment, the user simply generally places the controller on the wrist that requires no expertise or attention to specific sensor node placement. Further, the controller allows users to move freely as they would if they were not wearing the device.

### BRIEF DESCRIPTION OF THE DRAWINGS

The disclosure will be now described in more detail, with reference to the attached drawings, given as non-limiting examples, wherein:
Figure 1 illustrates the effect of the finer movement on the associated tendon.
Figure 2 is an illustration of the sensor module.
Figure 3 illustrates the effect of muscle contraction on the associated tendon.
Figure 4 is an illustration of wrist worn sensor.
Figure 5 is an illustration of the cantilever piezoelectric sensor.
Figure 6 is an illustration of the tendons in relation to finger movements.
Figure 7 is an illustration of the tendons in relation to the sensor module.
Figure 8 is an illustration of tendon tensions on the cantilever sensor.
Figure 9 is an illustration of an embodiment of the bottom view of the sensor.
Figure 10 is an illustration of an embodiment of the cantilever sensor.
Figure 11 is an illustration of a top view of an embodiment of the sensor.
Figure 12 is an illustration of the wrist-worn sensor built in in a watch strap.
Figure 13 is an illustration of the inclined sensors of the disclosure.
Figure 14 is a perspective view of an embodiment of a flexible sensor PCB.
Figure 15 illustrates top and lateral views of an embodiment of the PCB.
Figure 16 illustrates the relative position of the sensor to the wrist.
Figure 17 illustrates the effect of the silicone on low amplitude high frequency stresses.
Figure 18 illustrates the effect of finger pressure on the sensor.
Figure 19 illustrates the effect of high amplitude low frequency stresses on the sensor.
Figure 20 illustrates the information flow of a biometric signal on the sensor.
Figure 21 illustrates a 3D model of the main parts of the sensor module.
Figure 22 illustrates another 3D view of the sensor module.
Figure 23 illustrates the position of the sensor modules relative to the wrist.
Figure 24 illustrates the data processing elements of the sensor.
Figure 25 illustrates a process for classification of finger motions.
Figure 26 illustrates the measurement of tendon tensions by the sensor.
Figure 27 illustrates various signals resulting from specific finger movements.
Figure 28 illustrates signals from various sensors from a specific gesture.
Figure 29 illustrates an embodiment of the configuration of the sensor.
Figure 30 illustrates another embodiment of a sensor with two sensor modules.
Figure 31 illustrates a first embodiment of the sensor.
Figure 32 illustrates an embodiment of sensor module as part of a watchband.
Figure 33 illustrates a method for calibrating the sensor using a camera.
Figure 34 illustrates an embodiment of the electronics and sensor assembly.
Figure 35 illustrates another embodiment of the electronics and sensor assembly.
Figure 36 illustrates various cantilever sensor embodiments.
Figure 37 illustrates a graph of the signals for tapping the index finger for each of the four sensors.
Figure 38 illustrates a graph of the signals for tapping the ring finger for each of the four sensors.
Figure 39 illustrates a graph of the signals for flicking the index finger for each of the four sensors.

### DETAILED DESCRIPTION

A wearable controller as described herein is configured for measuring user muscles, tendons and bones position and activity on the wrist to interact with and control one or more computing devices. More specifically, the controller provides a wearable device having a linear array of cantilever piezoelectric sensors for detecting the movement of tendons in the wrist, an acquisition module, a signal processing module and a module for interacting with and/or controlling an external device. The external device may be a general purpose computing devices, software applications running on such computing devices, personal music players, physical devices coupled to a computing device, bionic devices, game consoles, televisions or other multimedia devices, virtual devices such as a virtual piano or virtual guitar implemented within a computing environment.

The controller is implemented in various form factors. In various embodiments, the controller may be implemented as a wristband, a wristwatch, or any other physical device or collection of devices worn by the user that has sufficient contact with the surface of the user's wrist skin to measure the activity of one or more of the user's tendons, muscles and other body tissues, and their combinations. Further, it should also be understood that a user can wear multiple controllers, with each such controller being used to interact with the same or a different computing device, application, or other attached device.

The voluntary movements made with the finger are generated by the muscle contraction in the forearm. These muscles transmit the force through the tendons. Therefore the tendons are subject to the tension forces and to the movements dictated by the skeleton mechanics. Every finger movement has a particular force and movement pattern. Every finger has its own particular set of tendons that move it, different from every other finger. The path of the tendon along the wrist is not rectilinear and is not strictly parallel to the forearm axis. The force vector that describes the dynamic of the force generated by the muscle contraction that moves the finger, is made of two components: one parallel to the forearm axis and one perpendicular. The tendon that pulls the finger moves the body tissues all around itself (body tissues comprising blood vessel, fat and skin).

The component of the force perpendicular to the forearm axis can be studied indirectly outside the wrist attaching a set of sensor to the skin at the wrist level and measuring the force needed to balance the perpendicular force vector.

The controller described herein measures in different ways all the movements in the wrist caused by finger gestures using an array of cantilever piezoelectric sensors. The measurements acquired from the sensors are combined and analyzed and classified by the controller and the control command sent to any electronic device.

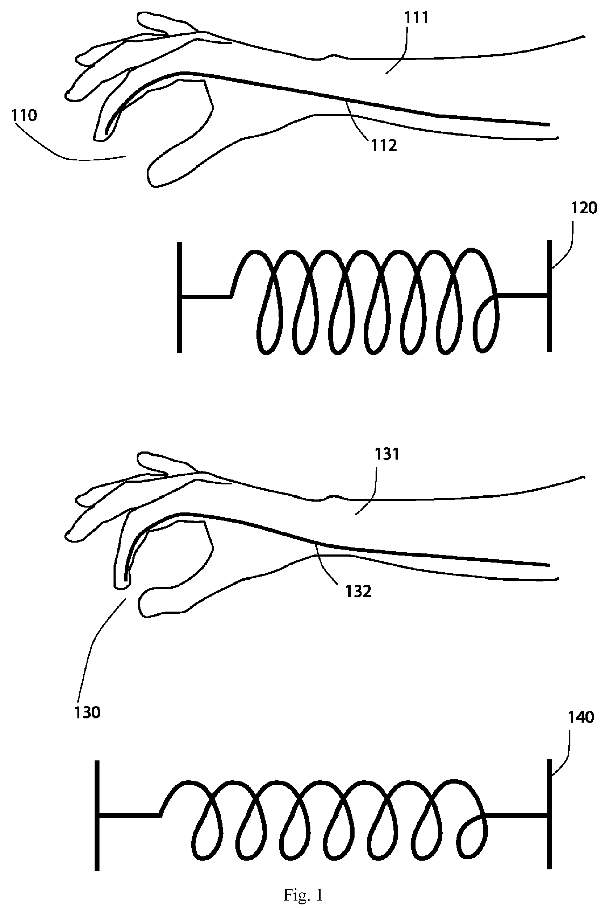

Figure 1 illustrates the effect of finger movements on the associated tendon. The tendon operates as an elastic element such as a spring. With fingers in a resting position 110, the tendon 112 in the arm 111 can be represented as a spring 120 without tension where no forces are applied to on both attachments of the spring. With fingers in a closing position 130, the tendon 132 is stretched just as the spring 140 is stretched as tension is applied. It is an object of the cantilever sensors in contact with the skin on the wrist to measure the tension applied to the tendon.

The cantilever piezoelectric sensors detect the movements of tendons associated with various finger gestures. A micro-controller or a microprocessor and the related electronics receive the signals from the sensors in order to process them and to send information, such as commands, to other devices.

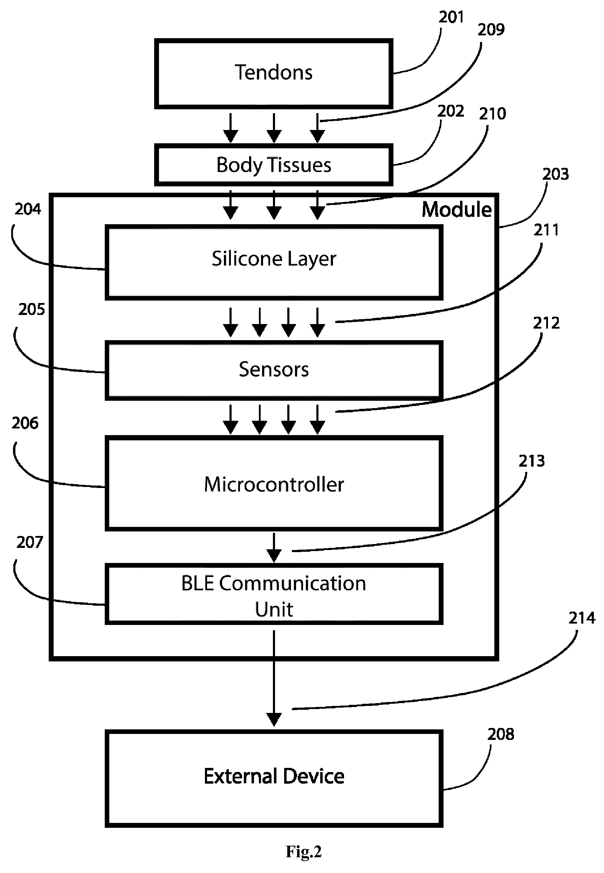

Figure 2 is an illustration of the different components of the sensor module. Movements of tendons 201 apply pressure on body tissues in the wrist 202. The sensor module 204 comprises a silicone layer 204 in which a plurality of cantilever sensors 205 are embedded. The sensors 205 acquire the pressure information 210 from the tendon movements and transmit the information to the microcontroller 206. The microcontroller 206 in turns transmits commands to a communication unit 207 which controls an external device 208. The above-described functional modules and components are employed for implementing various embodiments of the controller. As summarized above, the controller provides a unique device for measuring user hand activity associated with particular user gestures or motions for interacting with and controlling one or more computing devices.

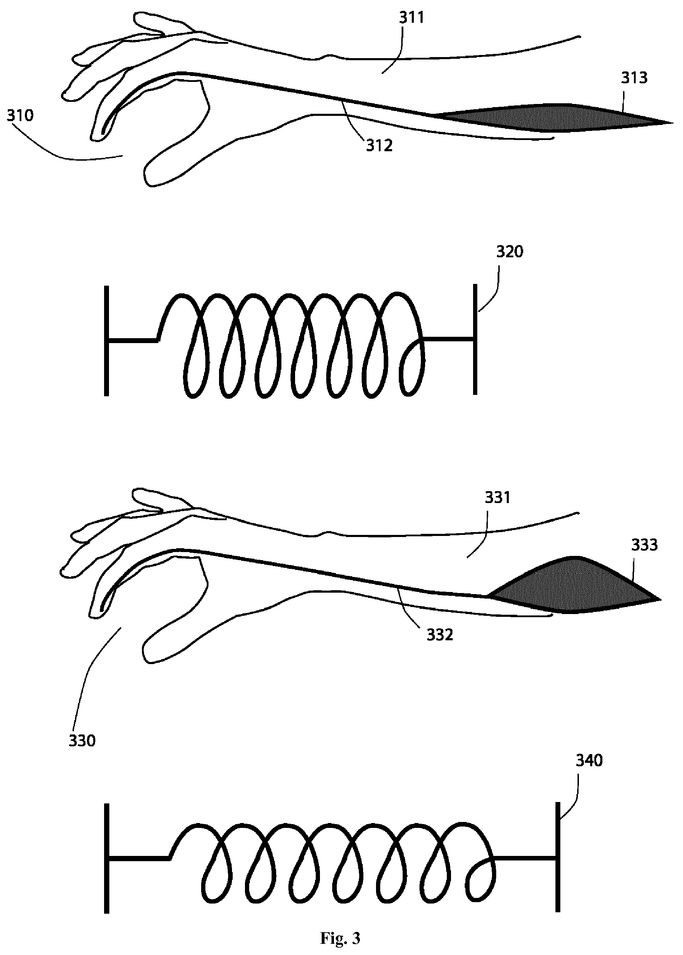

Figure 3 illustrates the effect of muscle contraction on the associated tendon. The tendon can be represented as an elastic element, such as a spring. In the first view, with fingers 310 at rest, the muscle 313 in the arm 311 is relaxed and applies no tension to the tendon 312. The tendon 312 in the arm 311 is in the resting status due to the finger position 310. In this case, the tendon can be represented as a spring 320 where no forces are applied to on both attachments of the spring, therefore without tension. In the second view the tendon 332 is stretched by the contraction of the muscle 333, whereas the finger position remains the same. This is similar to the situation where the spring 340 is stretched and the tension applied is higher. It is therefore an object the cantilever sensors, indirectly in contact with the tendons through the skin to measure the increase in tension of the tendons.

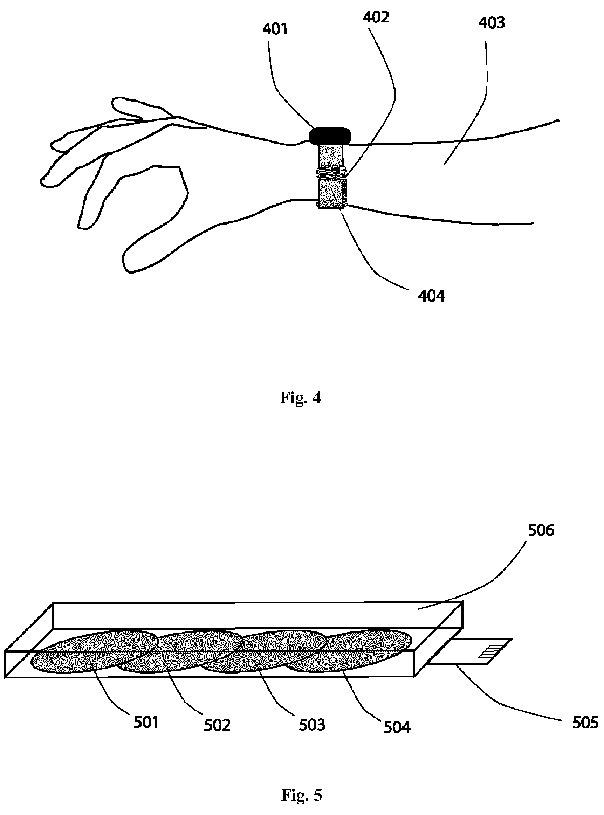

Figure 4 is an illustration of an embodiment of a wrist worn sensor. In this embodiment, the controller 402 is attached to the wristband 404 of a wristwatch 401.

Figure 5 is an illustration of an embodiment of the cantilever piezoelectric sensor module. In this embodiment, four partially overlapping cantilever piezoelectric elements 501, 502, 503 and 504 are embedded in a silicone module and electrically connected to a thin film PCB 505.

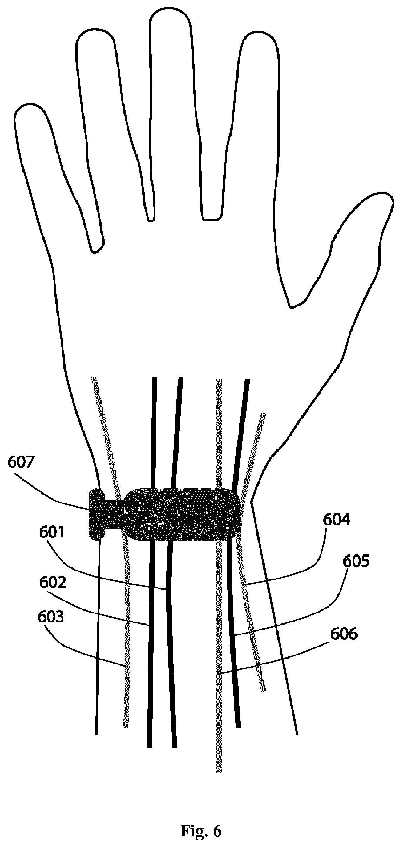

Figure 6 is an illustration of the tendons whose tension the controller is configured to measure. The six tendons are Flexor Digitorum Superficialis Tendon 601, Flexor Digitorum Profundus Tendon 602, Flexor Carpi Ulnaris Tendon 603, Flexor Carpi Radialis Tendon 604 Flexor Pollicis Longus Tendon in Radial Bursa 605 and Palmaris Longus Tendon 606. The most informative tendons relatively to the finger gestures are 601, 602 and 603.

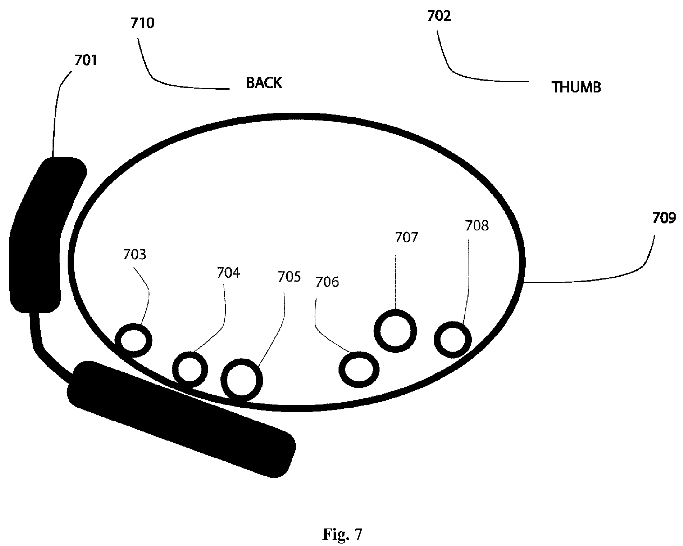

Figure 7 is an illustration of the tendons in relation to the sensor module. The controller module comprises two connected elements 701. This module takes advantage of the anatomical configuration of the wrist and in particular of the disposition of the set of tendons that go to the fingers. There are three main tendons that are directly correlated to the movement of the index, middle finger and annular respectively the Flexor Carpi Ulnaris Tendon 703, Flexor Digitorum Profundus Tendon 704 and the Flexor Digitorum Superficialis Tendon 705. The sensor element of the controller module is located proximally to these tendons so as precisely measure the tension forces applied to them in order to get information on the finger movements. The controller module 701 resides near these three tendons avoiding the direct contact with bones in order to be as comfortable as possible. The controller module is configured to take into account the variability of the position of the tendons within the wrist among different people. In a preferred embodiment, four cantilever sensors are positioned to pick up the force signals applied to the tendons.

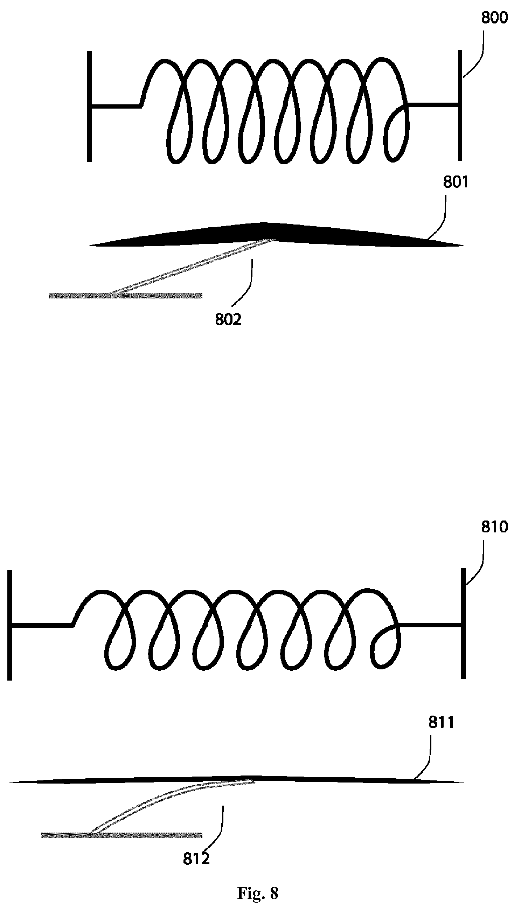

Figure 8 is an illustration of the effect of tension applied to tendons on the cantilever sensor. When the tendon 801 is relaxed, it can be represented as a relaxed spring 800. The cantilever sensor 802 will measure the tension of the tendon. If no tension is applied then no bending of the cantilever sensor occurs and therefore no signal is produced.
When the tendon 811 instead is stretched, it is squeezed and becomes longer and can be represented as a pulled spring 810. In this situation the cantilever sensor 812 is bent. The more tension is applied to the tendon, the more the sensor is bent and therefore more signal is produced.

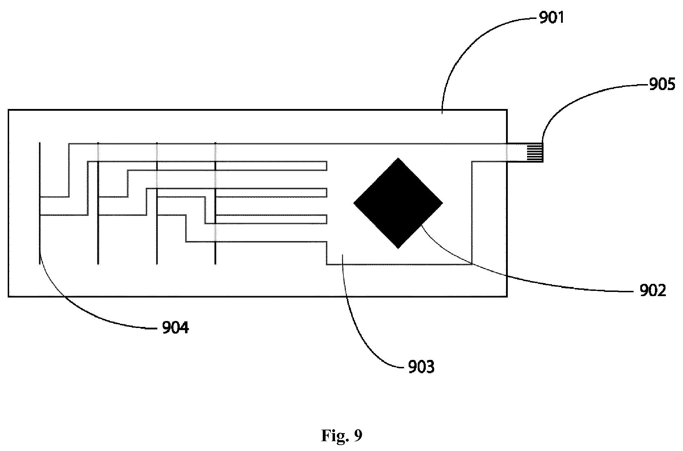

Figure 9 is an illustration of an embodiment of the bottom view of the sensor. In a preferred embodiment, the cantilever sensors 904 are embedded in a silicone element 901. A thin film PCB 903 is attached to the base of the silicone element 901 connects to the sensors 904 and includes a microcontroller 902 which processes the signal acquired from the cantilever sensors. A connector 905 attached to PCB 903 transmits the processed information to a communication module.

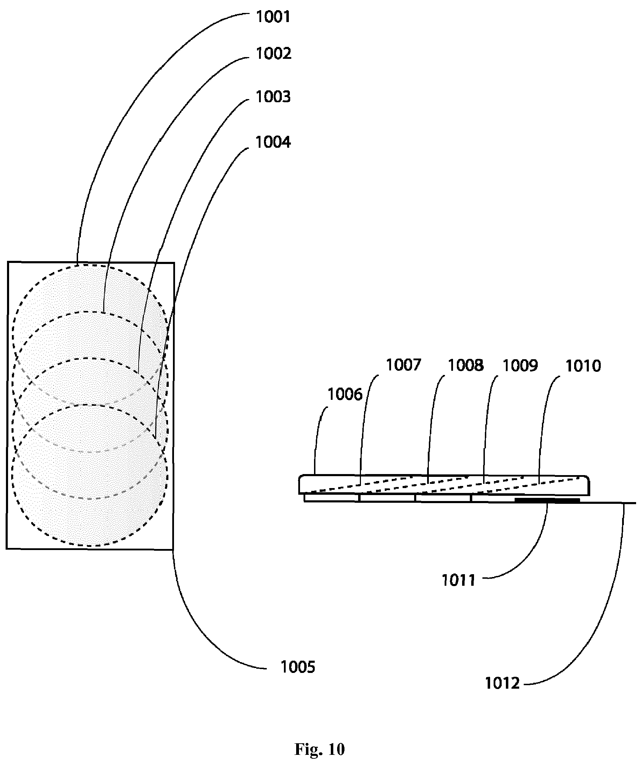

Figure 10 is an illustration of an embodiment of the cantilever sensor module. In a top view, a plurality of circular sensors 1001, 1002 1003 and 1004 are positioned in a linear pattern within a silicone matrix and are disposed so that the sensor areas overlap each other. In a lateral view, it can be observed that the sensors are cantilever at an angle in such a manner that only the tip of the sensor is attached to the PCB 1012.

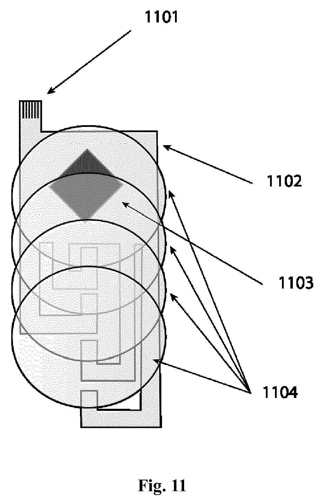

Figure 11 is an illustration of a top view of an embodiment of the sensor with the silicone removed. A plurality of sensors 1104 are positioned in a linear pattern, connected to a PCB 1102 comprising a microcontroller 1103 and a connector 1101.

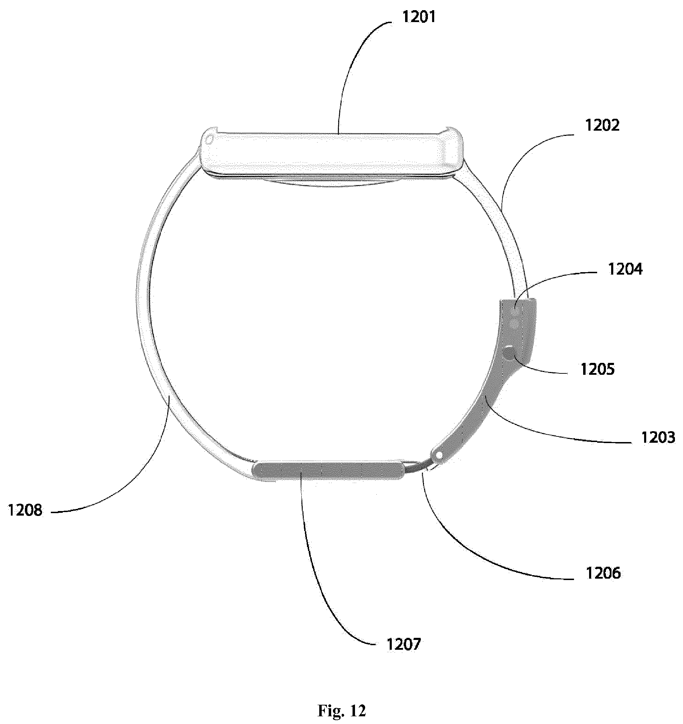

Figure 12 is an illustration of the wrist-worn sensor built in in a watch strap. A controller module comprising a bottom sensor and communication unit 1207, a connector 1206 and lateral battery module 1203 is attached to a watch face 1201 with two straps 1201 and 1208. In an embodiment, side pins for charging 1204 and a control button 1205 are disposed on the battery module 1203.

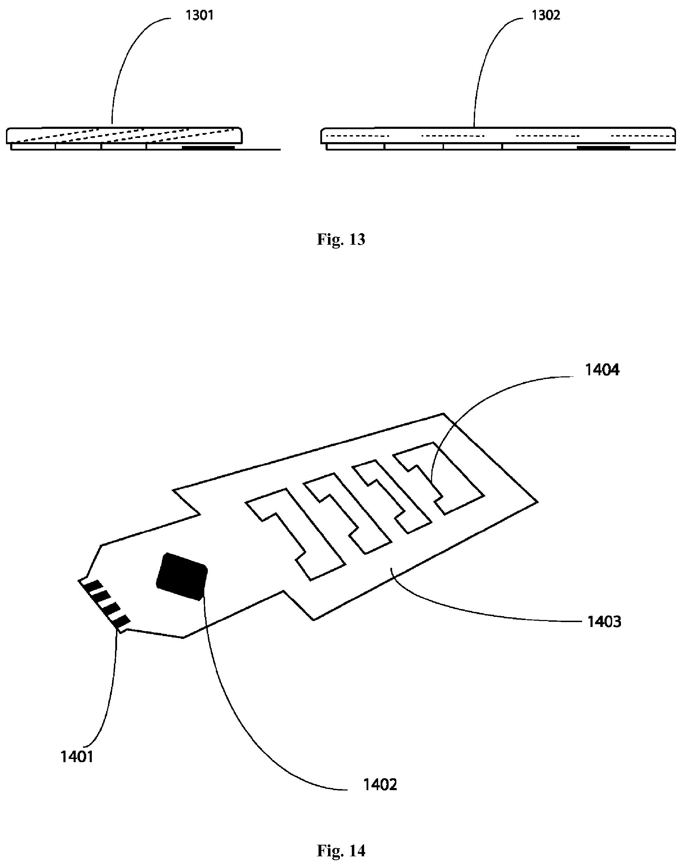

Figure 13 is an illustration of the effect of the inclined cantilever sensors of the disclosure. The piezoelectric sensors depending on where they are stressed they create different signals. In sensor arrangements of the prior art, large sensing areas create uncertainly in the location of the source of tension in the tendons and therefore in the measure of the actual movements of tendons, muscles and fingers. In the cantilever sensor design of the disclosure, only the tip of each sensor can be stressed, making the detection of the source of the signal highly accurate and more specific as to which tendon is being stressed. The cantilever and overlapping sensor layout design also allows for more sensors to be packed in a limited space increasing sensitivity of the sensor to small changes in tendon stresses.

Figure 14 is a perspective view of an embodiment of the electronic circuit of the sensor of the disclosure. The electronic circuit comprises a flexible thin film PCB 1403, a microcontroller 1402, a connector 1401 and angled wings 1404 that penetrate the silicone and to which the cantilever piezoelectric sensors are attached.

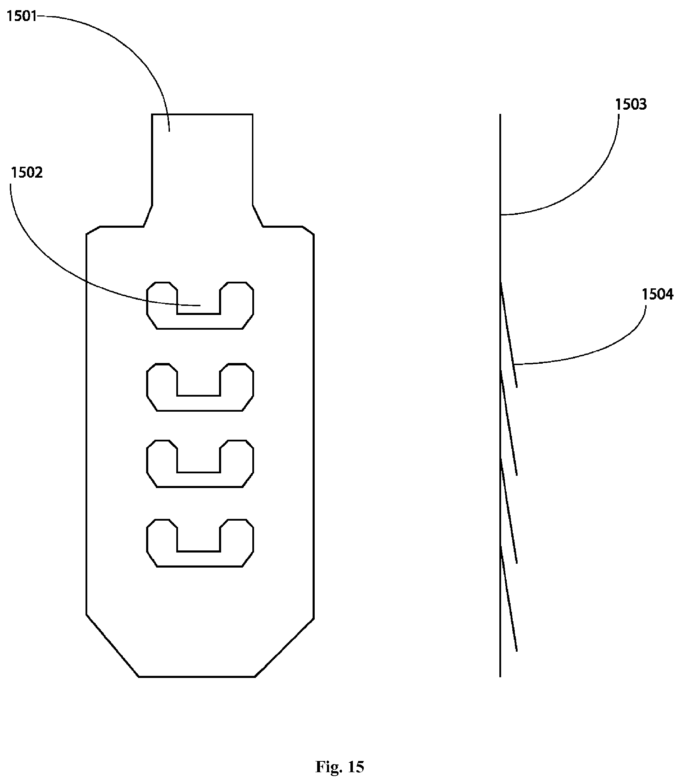

Figure 15 illustrates the angled wings 1502 in top view 1501 of the PCB and the same wings 1504 in side view 1503 of the PCB.

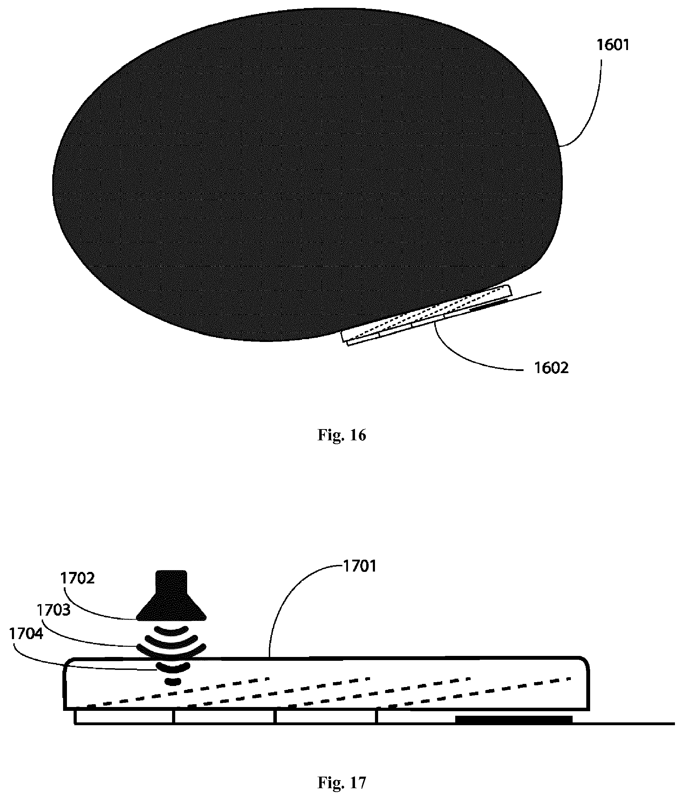

Figure 16 illustrates how the sensor module 1602 is placed against the wrist 1601 so as to optimally capture the tension applied to the wrist tendons.

Figure 17 illustrates the effect of the silicone material on low amplitude high frequency stresses. The silicone material 1701 filters out low amplitude high frequency mechanical stresses 1703 from source 1702 preventing them from form reaching the piezoelectric materials. In this case the pressure 1704 is damped and filtered and no signal is produced in the output.

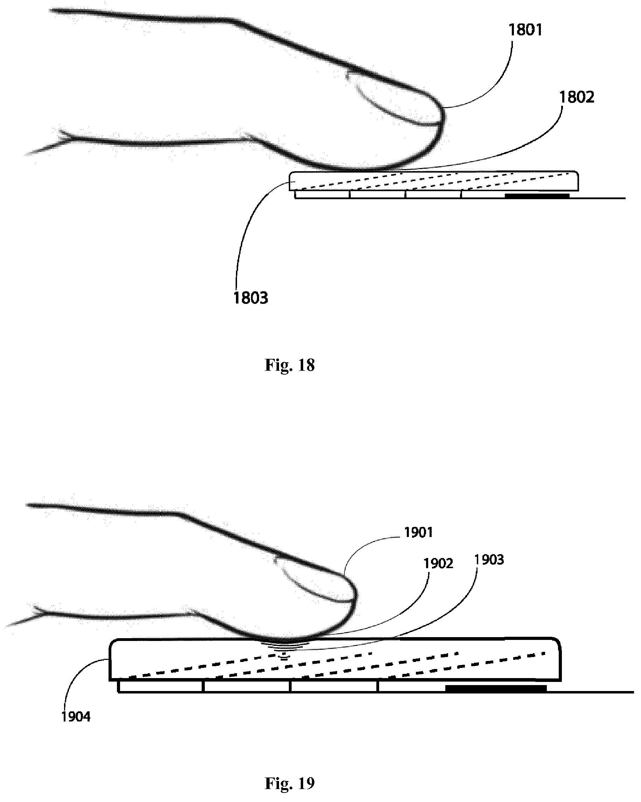

Figure 18 illustrates the effect of finger pressure on the sensor. Finger 1801 applies pressure on sensor module 1803. The plurality of cantilever sensors allows the sensor to detect the location 1802 of the applied pressure on the sensor 1803.

Figure 19 illustrates the effect of high amplitude low frequency stresses on the sensor. The sensors are embedded in a silicone material 1904, with a chemical composition, shape and size specified so as not to damp the signal generated by a source of high amplitude low frequency stress 1903 such as produced by pressure 1902 of a finger 1901.

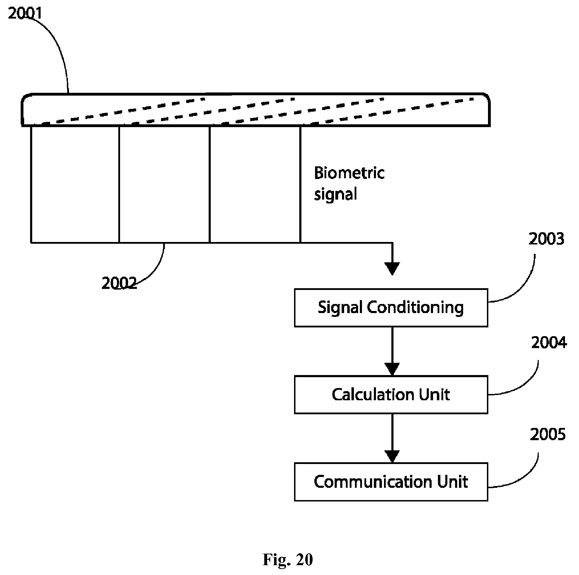

Figure 20 illustrates the information flow of a biometric signal on the sensor. An analog biometric signal 2002 will be transmitted from the sensor module 2001 to a signal conditioning unit 2003 where the signal amplitude will be adjusted before further transmission to a calculation unit performing analog to digital conversion and digital signal processing into commands before final transmission to a communication unit 2005 where the commands will be broadcast to an external device.

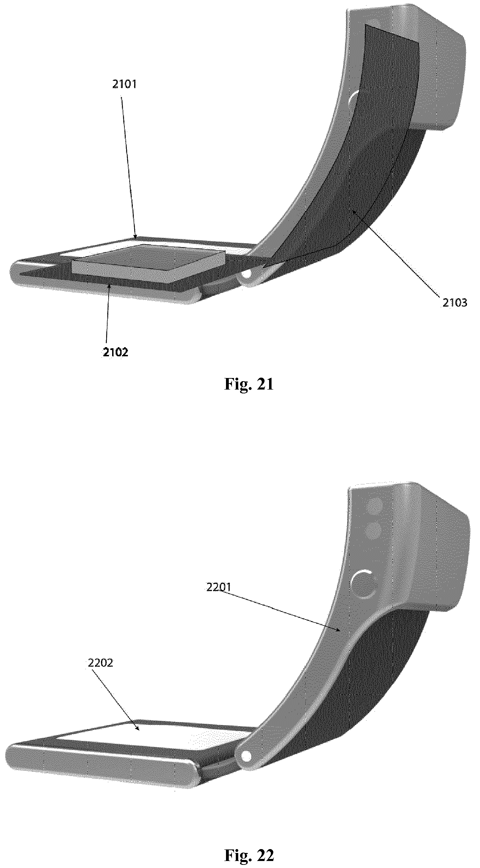

Figure 21 illustrates a 3D illustration of the main electronic parts of the controller. The controller comprises the sensor module 2101, the communication unit 2102 and battery 2103.

Figure 22 illustrates a 3D view of the controller with lateral module 2201 comprising the battery and external controls and the bottom module 2202 with the sensors and electronics. The sensor array within the controller can be automatically turned off in order to save power. The micro-controller can be set in rest-mode at the same time. This is particularly useful in wireless implementations of the controller where an onboard battery (replaceable or chargeable), fuel cell, photovoltaic power cell, etc., is used to energize selected sensor nodes and associated circuitry.

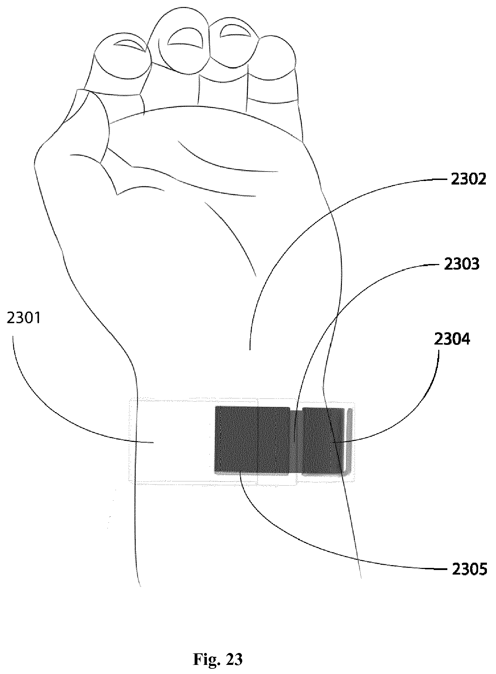

In order for a user to wear the module, it can be attached to an existing watchband or bracelet. This improves the usability of the controller, because a user is not requested to replace his wristwatch, since he can just attach the module to its own wristwatch and hide it under its watchband. Figure 23 illustrates the position of the lateral and bottom modules relative to the wrist. Bottom module 2305 and lateral module 2304 are attached together via connector 2303 and further held against the wrist 2302 with a watchband 2301.

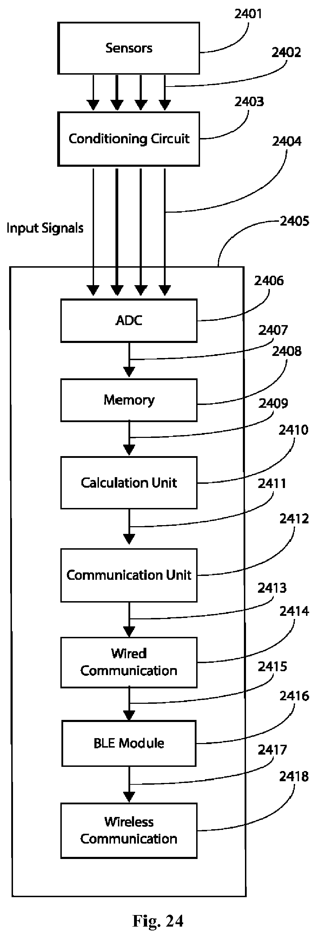

Figure 24 illustrates the data processing elements of the sensor. Pressure sensor 2401 comprises a silicone matrix with a linear array four piezoelectric sensors elements embedded inside. The following processing steps are performed by the controller:
a) the linear array produces four raw analog signals;
b) a conditioning circuit adjusts the raw signal to improve the signal to noise ratio;
c) an analog to digital converter unit converts the adjusted analog signal into a digital signal and produces elaborated results;
d) a memory module stores the input signals and the elaborated results;
e) a calculation unit runs a classification algorithm and produce results of the classification and diagnostic parameters;
f) a communication unit sends the results to an external device for communication.

In wireless implementations of the controller communication between the controller and one or more computing systems is accomplished via conventional wireless communications protocols such as, for example, radio frequency (RF) communications, infrared (IR) based communications, Bluetooth, etc. In this case, the controller includes one or more wireless transmitters, and optionally one or more receivers, for directly interfacing with one or more computing devices, or interfacing with one or more "hubs" that serve as intermediaries for interfacing the controller with one or more computing devices. In a preferred embodiment, a Bluetooth low energy module, able to broadcast wirelessly the information is used for communication with external devices.

It other embodiments of the controller, communications are implemented using wired connectors, such as, for example, by including an integrated USB that provides the power for the sensor nodes and provides a communications pathway between the controller and one or more external devices. As in the wireless embodiments, in wired embodiments, the controller communicates either directly with computing devices, or with those computing devices via an intermediary hub.

In addition, given the various wired and wireless configurations of the controller described above, it should be understood that hybrid embodiments using various elements of both the wired and wireless configurations are enabled. For example, in one embodiment, a power cable provides operational power, while wireless communications are then enabled by one or more transmitters/receivers integrated into, or coupled to, the controller. For example, in these types of hybrid embodiments, the power cable (e.g., a power cable connected to a transformer or other power source, or a USB power cable connected to a computing device or transformer, etc.) provides operational power to the controller, while the wireless transmitters/receivers provide communications between the controller and one or more computing devices or intermediary hubs within wireless range of the controller.

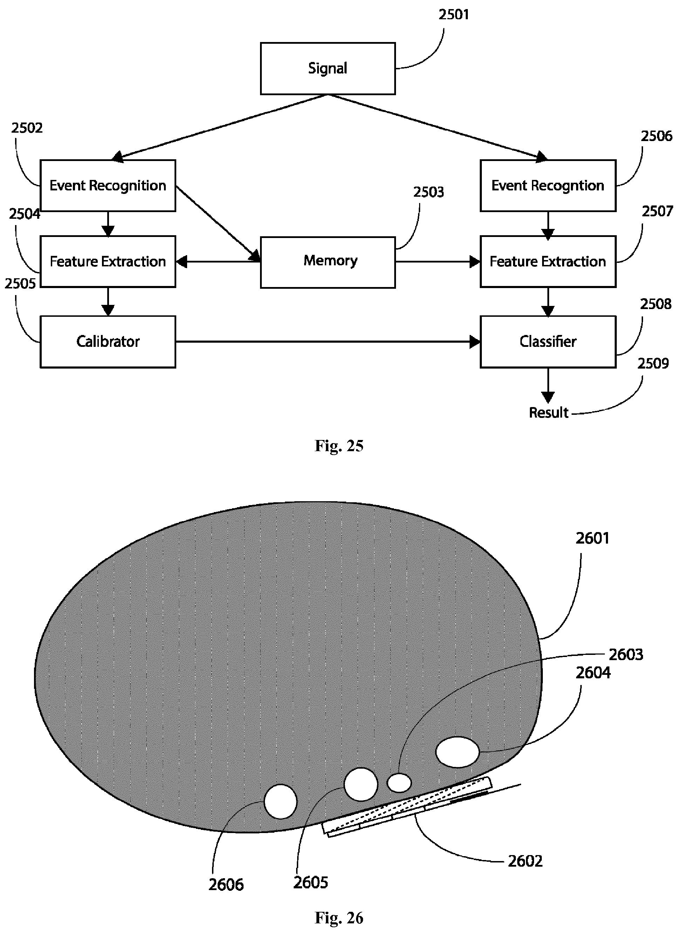

Figure 25 illustrates a process for classification of finger motions.
a) An event recognition module checks the signal stream until it detects that an event has happened. An event is anything different from the base noise.
b) When an event is detected, the module sends the registration of the event to a feature extraction module and goes back checking the signal stream.
c) The feature extraction module extracts meaningful parameters from the signals.
d) A calibration module gathers all the parameters coming from the feature extraction module and creates another set of parameters that allows a classifier module to classify the event.
e) The classifier module is able to recognize in which class the incoming parameters belong to. To do that it needs another set of parameters coming from the calibration module. The result of the classification is the name of the class.

Due to the wide heterogeneity of the human body, a calibration phase typically precedes the use of the device. The calibration is repeated periodically, in order to ensure the best performances. In the event that the classifier needs to perform a calibration, a calibration process is launched first. The memory module stores the old events recorded during the calibration. When requested by the feature extraction module, the memory module recalls the old events.

Figure 26 illustrates the measurement of tendon tensions by the sensor array. The sensor array while being indirectly in contact with the tendons keeps a dynamic equilibrium of the stresses applied to the wrist tendons. In the same way a finger can sense how much a guitar cord is stretched by touching the cord, the sensor array is constantly measuring the tension of the tendons and the changes in tension due to the finger movements and gestures. Each tendon creates pressure at different parts of the sensor causing a particular pattern of signals. The sensor array typically collects information from four main tendons including the Flexor Ulnaris Tendon 2603, the Flexor Digitorum Profundus Tendon 2604, the Flexor Digitorum Tendon 2605 and the Palmaris Longus Tendon 2606.

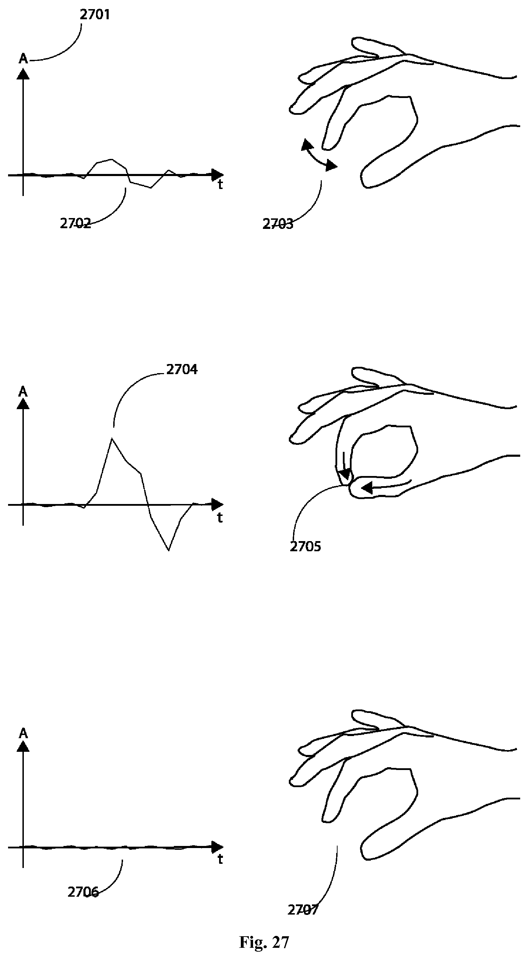

Figure 27 illustrates various signal patterns resulting from specific finger movements. The first graph 2701 illustrates the signal pattern 2702 from one of the sensors from the index finger moving up and down 2703. The second graph illustrates the signal pattern 2704 from one of the sensors from a pinching movement between the thumb and index finger. The third graph illustrates the signal pattern 2706 from one of the sensors from the fingers being still.

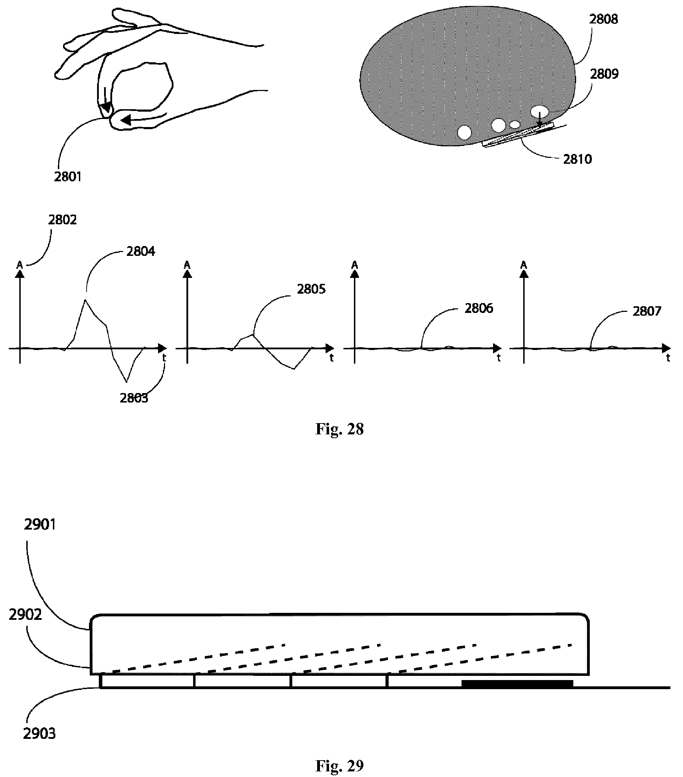

Figure 28 illustrates signals from multiple sensors in the array from a pinching gesture 2801 between the thumb and index finger. The four plots illustrate how each sensor in the sensor array 2810 is affected by the tendon tension forces. The wrist section view 2808 illustrates how a tendon applies pressure on a particular sensor 2809 of the sensor array 2810.

Figure 29 illustrates an embodiment of the configuration of the sensor. A first layer 2901 made from an elastomeric material such as silicone rubber filters out low amplitude signals and provides comfort against the skin. When the sensor array touches the wrist, the elastomeric material blocks noise from reaching the sensor array and bends slightly and changes shape to ensure close contact with the wrist. The second layer comprises piezoelectric elements embedded in an elastomeric material providing a robust sensing structure (the piezoelectric material is made of ceramic and is really fragile alone). The third layer consists of a thin film PCB typically made from a thin flexible plastic or metal foil material. The three layers combine to form a compact and flexible sensor module.

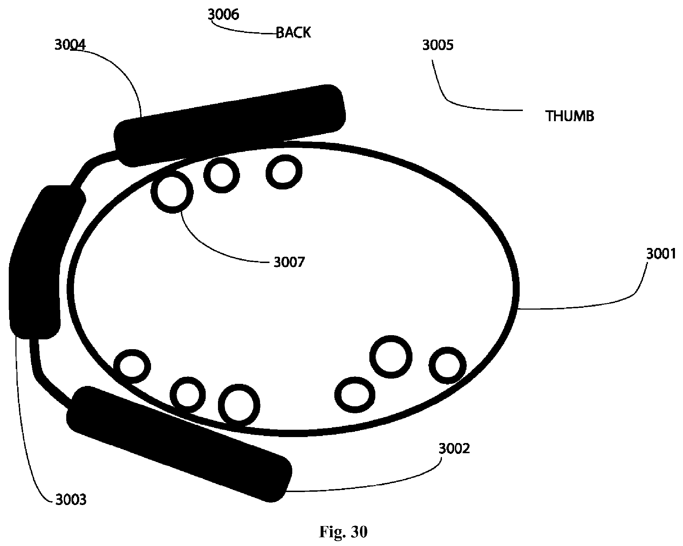

Figure 30 illustrates another embodiment of a controller with two or more sensor modules, one module for the wrist tendons 3002 and a second sensor module 3004 for the tendons controlling finger extension. A third lateral sensor 3003 may be provided as well for additional accuracy.

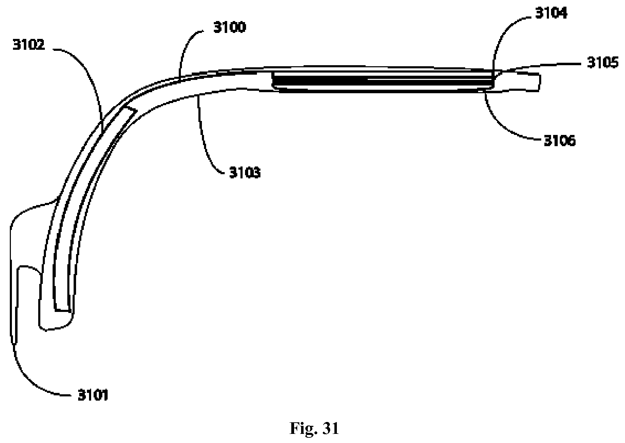

Figure 31 illustrates a first embodiment of the sensor module. A battery 3102 is attached to a first electronic circuit 3104 comprising a Bluetooth module, Bluetooth antenna and other electronic components, via connection 3100. Sensor 3106 is attached to electronic circuit 3104 via a thin film PCB 3105. A clip 3101 allows the sensor module to be attached to a watchband or fitness band. The whole sensor may be embedded in a silicone or other elastomeric material 3103 for water resistance and greater comfort.

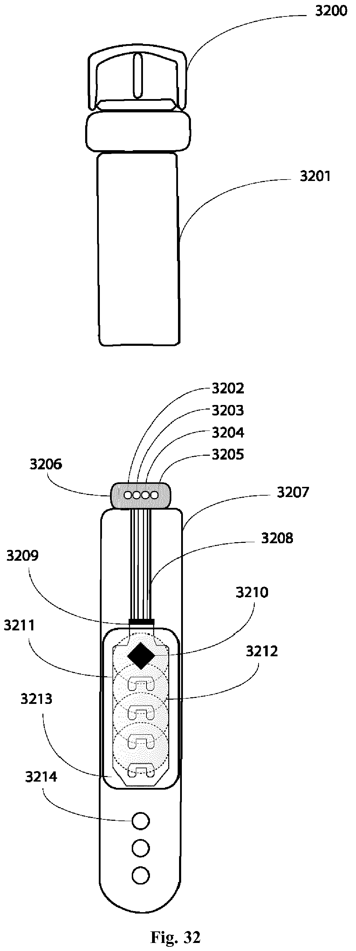

Figure 32 illustrates an embodiment of sensor module as part of a watchband. The sensor module comprising the sensor array 3212, thin film PCB 3211 and microcontroller 3210 embedded in elastomeric material 3213 is directly connected to a smartwatch via a wired connection 3208 eliminating the need for a separate power supply. The sensor and electronics are directly mounted on one side of a watchstrap 3207. A custom connector 3206 to the smartwatch comprises a ground connection 3202, a power supply connection 3203 and communication channel connections 3204 and 3205.

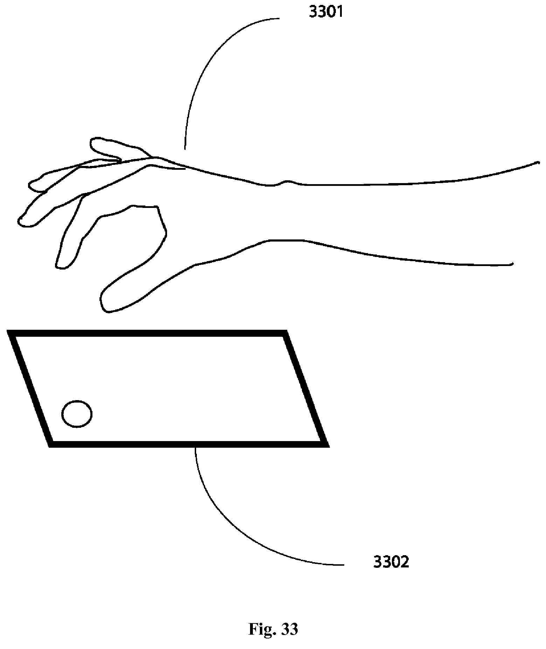

Figure 33 illustrates a means of calibrating the sensor module using computer vision. Such a calibration method can gather more information on how the user is performing the gestures and on the user hand characteristics taking advantage of another device's camera, such as a smartphone camera in order to monitor the hand while performing the calibration.

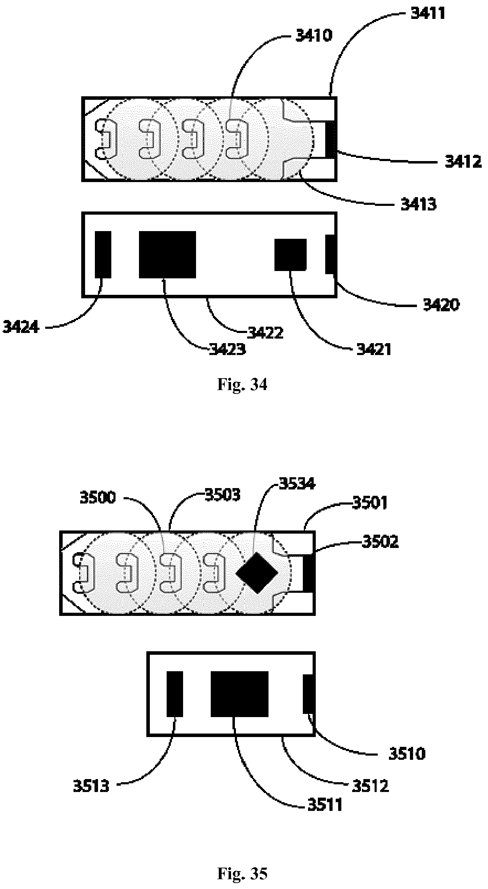

Figure 34 illustrates an embodiment of the electronics of the sensor module. Flexible PCB 3410 connects piezoelectric elements 3413 embedded in elastomeric material 3411 to connector 3412. Microcontroller 3421, Bluetooth chip 3423, Bluetooth antenna 3424 are connected to rigid thin PCB 3422 and connector 3420.

Figure 35 illustrates another embodiment of the sensor and electronics assembly with microcontroller 3534 mounted on the thin film flexible PCB 3530 together with sensor array 3533 embedded in elastomeric material 3531 and attached to connector 3502. Bluetooth chip 3511, Bluetooth antenna 3513 and electronics connector 3510 are mounted on the rigid thin PCB 3512.

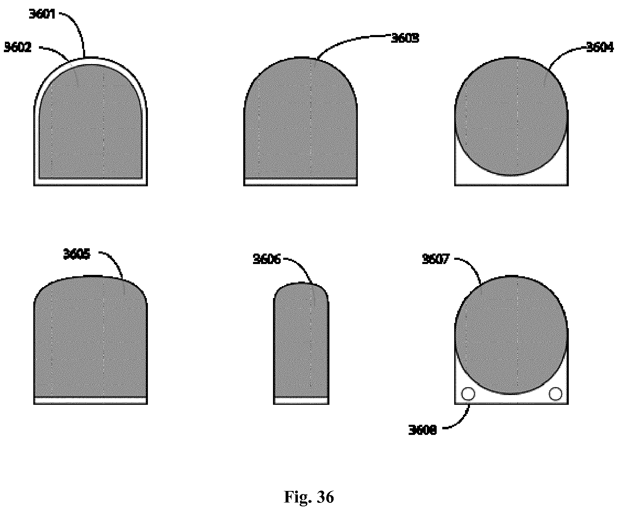

Figure 36 illustrates various cantilever sensor embodiments. A key feature present in all these drawings is the straight line on the bottom. This shape provides strong stability to the PCB layer underneath, higher precision for assembly and easier manufacturability. All the sensors are made of two layers: a metal layer and the piezoelectric ceramic layer. Typical width of the sensors is 12 mm although other widths are contemplated by the disclosure. The 3601 sensor version is similar to the off-the-shelf piezoelectric sensor, preserving a border of plain metal that allows a good placement of the glue that attaches the two layers. The 3603 sensor version comprises an enlarged ceramic plate, for greater sensitivity increases. The 3604 sensor version retains the usual shape of the ceramic plate attached to a larger and more stable metal plate. The 3605 sensor version is a variation of 3603 where the curvature on the top corners is smaller, allowing for a wider ceramic plate. The 3606 sensor version is half the width of the 3605 sensor width of 12 mm. This version has a lower sensitivity for the same bending angle, although the elongated shape allows for greater bending resulting in the same signal amplitude for the same pressure applied. Its smaller dimension allows for a greater number of sensors in a given size array improving the spatial resolution of the sensor array. The 3607 sensor is a variation of 3604 sensor. The two holes along the bottom make the sensor easier to handle during manufacturing and provide a more stable attachment to the final device.

As discussed herein, the sensors of the controller are applied coarsely, without an expert present to ensure precise placement. For example, in the aforementioned wristband configuration, an end-user attaches the armband on the wrist, such that sensors are located next to the wrist skin.

Given this approach, the basic process of "installing" the controller can be implemented in a number of user-friendly ways. In an embodiment, initial positioning of the controller is accomplished using a process such as the simple three step process illustrated below: 1) The user puts the wristband, wristwatch, or other controller in a coarsely approximate location where the device is intended to be placed. For example, would be coarsely placed somewhere on the users wrist. The system would then be activated or turned on (unless the system was already activated or turned on); 2) The user would then make coarse manipulations to the initial positioning of the device, such as, for example, rotating the wristband, while receiving simple feedback about signal quality (such as a simple "meter" on a computer screen, a sound emanating from the device, or speech cues to direct the user with respect to specific motions); 3) Finally, the user would make fine adjustments to the position or orientation of the device (e.g. rotate and/or move the position of the controller) until a simple goal is achieved, such as "meter goes above level 5," "sound stops", "vibration stops", etc.

In various embodiments, the feedback provided to the user during this simple adjustment process is visual (e.g., a bar or meter on a computer screen, on a portable music player, or on a small on-board LCD or series of one or more LEDs or lights), auditory (e.g., a noise that gets quieter as signal quality increases, or a voice saying "keep turning, keep turning, perfect!"), or haptic (e.g., the controller vibrates or electrically stimulates one or more areas of the user's skin while the user should continue to adjust the device and stops vibrating or electrically stimulating the user's skin when the signal quality is adequate.

The wrist-worn controller provides HCI capabilities based on signals generated by the body in response to the contraction of one or more tendons connected to the fingers. As such, it should be clear that the controller is capable of being used for any of a number of purposes. For example, these purposes include interaction with conventional application such as interacting with a computer operating system by moving a cursor and directing simple object selection operations (similar to using a computer mouse to select an object), wired or wireless game controllers for interacting with game consoles or with video games operating on such consoles, control of pan-tilt-zoom cameras, interaction with home automation systems such as audio, video, or lighting controls, etc.

Other obvious uses for the controller include local or remote control of robots or robotic devices, such as, for example, using a glove with embedded sensor nodes on the wrist to control a remote robotic hand wielding tools or medical instruments.

The controller can be fitted with an additional accelerometer in order to measure the movements of the whole hand in the space, in order to have more information to send.

The controller described herein is operational for interfacing with, controlling, or otherwise interacting with numerous types of general purpose or special purpose computing system environments or configurations, or with devices attached or coupled to such computing devices. For example the wristwatch can act as a "hub" in the case, as a wireless intermediary between one or more of the sensor nodes and a second device.

In one embodiment, the controller communicates with a computing device. Such computing devices include, but are not limited to, personal computers, server computers, hand-held computing devices, laptop or mobile computers, communications devices such as cell phones and PDA's, multiprocessor systems, microprocessor-based systems, set top boxes, programmable consumer electronics, network PCs, minicomputers, mainframe computers, video media players, in-vehicle computing systems (e.g., automotive computer system), etc.

As noted above, computing devices such as those described herein operate either in response to user gestures recognized via one or more controller. However, in various embodiments, such computing devices also provide computing power for operations such as the initial calibration. In addition, such computing devices may also act as hubs or intermediaries to facilitate communications between the controller and one or more other computing devices or attached mechanisms. In general, such computing devices include at least some minimum computational capability along with some way to send and receive data. In particular, the computational capability is generally given by one or more processing unit(s), and may also include one or more GPUs. Note that that the processing unit(s) of the general computing device of may be specialized microprocessors, such as a DSP, a VLIW, or other micro-controller, or can be conventional CPUs having one or more processing cores, including specialized GPU-based cores in a multi-core CPU.

In addition, the computing device may also include other components, such as, for example, a communications interface. The computing device may also include one or more conventional computer input devices (such as a microphone or microphone array for receiving voice inputs). The simplified computing device may also include other optional components, such as, for example one or more conventional computer output devices (such as audio and/or video output devices). Finally, the computing device may also include storage that is either removable and/or non-removable. Note that typical communications interfaces, input devices, output devices, and storage devices for general-purpose computers are well known to those skilled in the art, and will not be described in detail herein.

After band-pass filtering the signals in case the amount of data is too great, it can be analyzed by a real-time PCA. The PCA permits to reduce the amount of data and focus on the relevant signals. The signals are then analyzed by a feature extractor. The feature extractor analyzes the signals in order to obtain a set of features that robustly describe the signals and that can be compared with other signal features coming from other finger movements and gestures. The comparison is usually made in order to classify the signal and recognize the associated finger gesture. The feature can be a time domain feature (amplitude, ratio between the signal amplitude and other prerecorded signals amplitudes, number of lobes, number of zero-crossings, time length of each lobe, time length of each movement, correlation with other pre-recorded signals, difference between the signal and other pre-recorded signals). The feature can be a frequency domain feature (power of the spectrum, power of a range of frequencies, ratio between amplitude of certain range of frequencies, wavelet features).

A preferred system for power management is described as follows. The embodiment is normally set in sleeping mode, the signal acquisition is not active, the microcontroller is set in low power consumption. The microcontroller wakes up from the sleeping mode thanks to an external signal triggered by a mechanical button, which is preferably placed in the part of the device which is in contact with the wrist. When the user's wrist flexes, said button is pressed and activates the signal. The flexion movement of the user's wrist increases the pressure of the device itself onto the wrist skin, triggering the activation of the button. With said power management system, two problems are prevented: high power consumption and accidental gestures that the user might otherwise perform involuntarily which could cause wrong commands.

The foregoing description of the controller has been presented for the purposes of illustration and description. It is not intended to be exhaustive or to limit the claimed subject matter to the precise form disclosed. Many modifications and variations are possible in light of the above teaching. Further, it should be noted that any or all of the aforementioned alternate embodiments may be used in any combination desired to form additional hybrid embodiments of the controller. It is intended that the scope of the disclosure be limited not by this detailed description, but rather by the claims appended hereto.

### CALIBRATION

In general, it is assumed that users of the controller will not place the device (or individual sensor nodes) in exactly the same place relative to specific tendons each time that the user wears the controller. Further every individual has a different anatomy. One aspect of the controller is the capability to rapidly calibrate itself to any individual wearer.

Calibration can be accomplished in various ways. For example, in one embodiment, calibration is accomplished by connecting the controller to a main station such as a computer or a smartphone, with the calibration software program installed. The software program asks the user to make some finger gestures while wearing the controller, and collects the parameters useful for the device to recognize the gestures. Once finished with the calibration, the controller receives the parameters and thus is ready to work.

Note that in training or retraining the classification system, given the limited number of muscles involved in such gestures, in various embodiments, the classification system is trained or calibrated by using only a subset of recognized gestures or motions in order to find matching points from previously built models.

Further, in various embodiments, this calibration is continually or periodically performed as the system observes the user's actions. Note that periodically or continuously performing the calibration serves at least two purposes. First, repeating the calibration process may help to further refine the gesture model, and second, repeating the calibration process will help to adjust for minor positional movements of the controller on the user's body.

In addition, since the controller is worn by the user, calibration data can be collected even when the user is not actively engaged in using the controller for HCI purposes. This additional calibration data collection allows the system to statistically model likely gestures or movements, and given enough time, the system can infer the gestures or movements that the user is performing.

The controller presents a micro-controller and related electronics able to read the sensors signal, filter it and analyze it in order to perform the gesture recognition and classification. The micro-controller receives the parameter for the classification during the calibration. The calibration can be made by the micro-controller itself or by another computer device connected to the micro-controller.

The purpose of the signal processing unit is to classify the input signals. The classification is based on a set of signals given during calibration. The calibration involved in the training of the classifier has three different phases.
- First phase called Hard calibration
- Second phase called Soft Calibration
- Third phase called Continuous Calibration

During hard calibration the user is asked to repeat 4 times each gesture. This process is long, takes from 30 to 60 seconds and is very accurate. During soft calibration the user is asked to repeat each gesture just once. It uses a hard calibration stored in the memory. It updates the stored hard calibration in order to adjust the parameters. It is faster than the hard calibration, and takes less than 10 seconds to perform. During continuous calibration as the classifier runs and recognizes a gesture, the same gesture is also used to recalibrate the classifier algorithm itself. This continuous calibration permits to take into account minimal shifts of the module from the initial position. Shift after shift the module can change the position on the wrist when used with a watch. This calibration avoids asking the user to repeat a soft/hard calibration. This calibration is completely automated and does not involve the user.

In an alternative calibration the user is asked to move the finger in front of a camera that recognizes the movement of the finger tips and then autocalibrates the algorithm based on the finger movements. This method releases the user from following the usual calibration process.

The usual calibration process requires the user to follow a set of instruction on how to perform the gesture that sometimes might be mis-interpreted by the user. The algorithm is hence divided into two main parts: the calibration and the execution. The calibration starts if the "Calibration needed" block receives a value that is lower than a predefined threshold. The first time this algorithm runs the the result is always positive because the received value "quality of the classification" is zero. Therefore the Event recognition of the calibration side is active and it analayses the input signal waiting to receive a signal that overcomes a certain threshold that triggers the recognition. The input signal is stored inside this block in a FIFO memory. When the event recognition block is triggered, it moves the FIFO memory to the output. It also counts how many times the event is triggered. The FIFO contains a section of the input signal flow, called windowed signal. This windowed signal is stored in the memory in a slot decided by the count value.

When the count value reaches a certain predefined value "end calibration", the Feature extraction block is triggered. The feature extraction block goes to the memory and analyses all the recorded signals and returns a set of template values that are saved directly in memory. These values gather the significant information extracted from the stored signals. These information are then used to train the classifier.

The Classifier training block then returns an index that represents the quality of that calibration. If this index is above a predefined threshold, the execution phase can start. The first step of the execution phase is the event recognition block, identical block to the one in the calibration phase except for the lack of the counter. The event recognition receives and stores a signal in a FIFO memory. When the signal overcomes a predefined threshold, the module is triggered and it returns the content of the FIFO memory. This set of data goes to the feature extraction module, which purpose is to extract the most characteristic features of the signal and avoid the useless information. This part of useful information is finally sent to the trained classifier that is capable of recognizing which class the input signal belongs to. The classes are defined during the classification training. The final result of the classification is the name of the class. It also returns the index of the quality of the classification.

### GESTURE RECOGNITION

In various embodiments, the user is provided with mechanisms for performing user-defined gestures or sequences of gestures, and then assigning particular actions to those gestures, either from a pre-defined list of actions, or from user defined actions or macros. In this case, the training described above is the same, with the difference simply being the particular command or macro that is being mapped to the predefined or user-defined gesture.

In various embodiments, an important finger gesture such as the action of tapping a finger against the thumb will be provided by protocol. Such gesture is recognized by the sensor array. It detects that the gesture has been performed, and the position of the tendon involved in the action, in order to identify the finger movement. These pieces of information are given by a trained classifier after a brief signal filtering. The classifier is trained during the calibration.

In order to apply SVM algorithm for gesture recognition, some elements have to be set:
1- features
2- dimension of the dataset
3- stopping condition
Different features have been analyzed related to different approaches: differences, time domain features. Better results has been obtained through differences i.e. difference between signals after alignment using a convolution approach. Each feature represents the difference between a signal and a template related to the same microphone. So the minimum number of features is 4 equal to the number of sensors. A binary classification makes it necessary to define two different templates: one for each target. In such a way, the number of features is increased to 8.

Concerning the dimension of the dataset, minimum dimension which do not modify the classification quality is 4 repetitions for each class, so that features matrix has 8 rows and 8 columns. From column 1 to 4 differences are calculated with respect to a template of the first gesture. From column 5 to 8 features are calculated as differences from second gesture as template. From row 1 to 4 signals are related to the first gesture, while from row 5 to 8 are related to the second gesture. So, a feature matrix with 4 sub-matrixes is obtained. In fact, differences performed within the same gesture are lower when compared to difference between different gestures.

Such results are obtained in an analysis with two gestures in comparison with the results of the algorithm based on differences implemented on Arduino.

In terms of stopping condition, it is defined through the tolerance of each SVM which is an initialization parameter. Once the training has been performed, if the same training examples are given as inputs, it could be verified that they do not give outputs symmetrically distributed around zero. It happens most frequently with a small dataset rather than with more examples. So the sign of the output is not evaluated around zero. A new threshold is calculated as the mean value between outputs from a single SVM when it receives as input the training set.

### SIGNAL PROCESSING

Some analyses can be performed on the raw sensor signals to determine gesture-recognition system. These analyses are generally computationally simple, and are thus suited to being performed on the microprocessors that are built in to each wireless sensor node or into an integrated device such as the aforementioned wristband. However, as discussed above, such processing of raw signals can also be on a downstream receiver or processor during an initial high-power calibration phase. Examples of raw-signal analyses that can provide indications of signal relevance include measures of RMS amplitude of the finger-generated signals and measured power bands.

Signals within a known range of amplitude are most likely to be informative. In this case, a very simple logic test to determine whether a measured signal is within a simple range can be included in the individual sensor nodes, such as, for example, by adding a simple logic gate to the analog to digital converter or to the digital signal processing module. Similarly, an analysis of individual frequency bands of measured signals can also be performed using very simple computational capabilities. For example, in the case that a particular signal where one or more individual frequency bands falls outside a "reasonable" or expected range in known frequency bands are unlikely to be informative. Gesture analysis has underline that minimum sample frequency for the signal is about 200 Hz in the time domain while it is about 800 Hz for the derived signal. Both signals bring to equal results in terms of accuracy in gesture recognition. The only difference is represented by the buffer size. In fact, in the first case, at least 150 samples are needed while the derived signal requires 100 samples. The main benefit in using derived signal is related to use of few samples. As a consequence, only gestures with a fast variation in the magnitude related to each sensor can be recognized.

### SIGNAL EXAMPLES

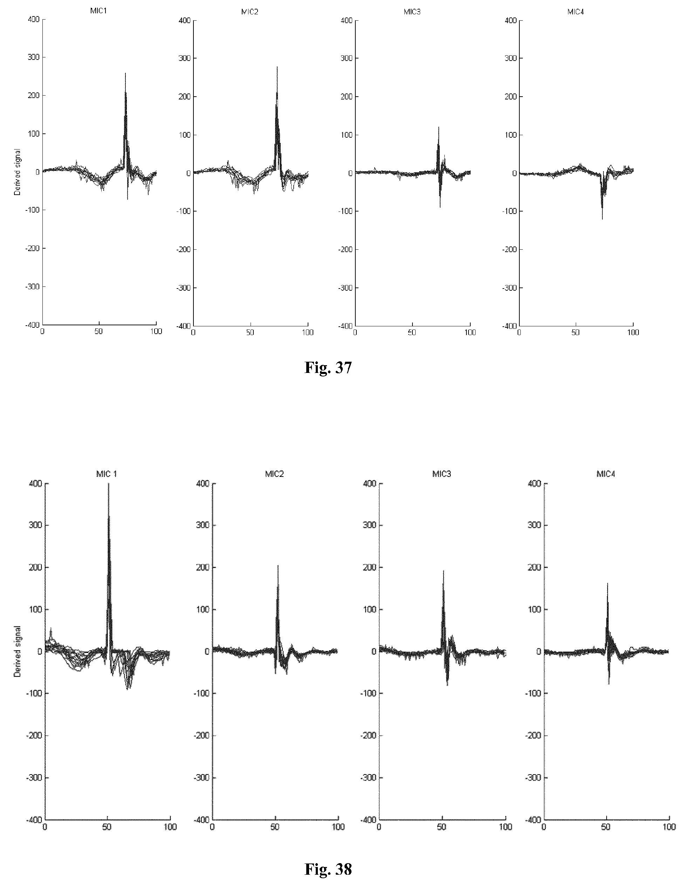

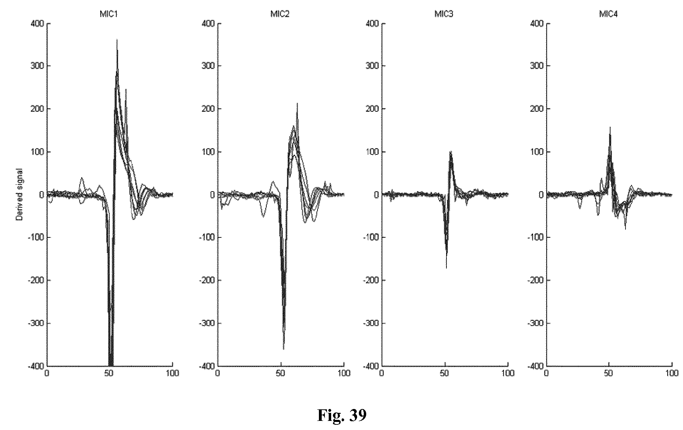

The Following graphs show three gestures which can be recognized with high accuracy through a SVM based on 3 gestures and features based on the differences involving the derived signal. Figure 37 illustrates a graph of the signals for tapping the index finger for each of the four sensors. Figure 38 illustrates a graph of the signals for tapping the ring finger for each of the four sensors. Figure 39 illustrates a graph of the signals for flicking the index finger for each of the four sensors.

Feature matrixes are reported below:
1) SVM1: tapping index (1) VS tapping ring finger (2)

**feat_matrix1_2 =**

| | | | | | | | |
|---|---|---|---|---|---|---|---|
| 0.4141 | 0.2749 | 0.5097 | 0.3342 | 0.8855 | 0.8417 | 1.0000 | 0.6329 |
| 0.3365 | 0.3008 | 0.6740 | 0.2497 | 0.9227 | 0.8402 | 1.0000 | 0.7243 |
| 0.4217 | 0.3470 | 0.6115 | 0.3768 | 1.0000 | 0.9446 | 0.9632 | 1.0000 |
| 0.4096 | 0.3182 | 0.5400 | 0.3752 | 0.9369 | 1.0000 | 1.0000 | 0.9177 |
| 1.0000 | 0.7955 | 0.8323 | 0.7154 | 0.6900 | 0.2657 | 0.2770 | 0.5050 |
| 1.0000 | 0.9049 | 0.9932 | 0.7662 | 0.5258 | 0.2769 | 0.2729 | 0.3981 |
| 0.9527 | 1.0000 | 0.9399 | 0.8243 | 1.0000 | 0.3831 | 0.4399 | 0.5798 |
| 1.0000 | 0.9832 | 1.0000 | 1.0000 | 0.7608 | 0.3542 | 0.4191 | 0.6541 |

2) SVM2 tapping index (1) VS flick (3)

**feat_matrix1_3 =**

| | | | | | | | |
|---|---|---|---|---|---|---|---|
| 0.1180 | 0.1786 | 0.1438 | 0.1976 | 1.0000 | 0.9643 | 0.9263 | 0.8468 |
| 0.0991 | 0.2021 | 0.1966 | 0.1526 | 1.0000 | 1.0000 | 1.0000 | 1.0000 |
| 0.1068 | 0.2005 | 0.1533 | 0.1980 | 1.0000 | 0.9598 | 0.9932 | 0.8216 |
| 0.1078 | 0.1911 | 0.1407 | 0.2049 | 1.0000 | 0.9435 | 0.9329 | 0.9430 |
| 1.0000 | 0.8719 | 1.0000 | 0.8268 | 0.1586 | 0.2474 | 0.2176 | 0.3160 |
| 1.0000 | 1.0000 | 0.8214 | 1.0000 | 0.3333 | 0.4002 | 0.3821 | 0.6115 |
| 1.0000 | 0.9389 | 0.9411 | 0.7452 | 0.4535 | 0.5030 | 0.5294 | 0.7724 |
| 1.0000 | 0.7426 | 0.9175 | 0.6921 | 0.3121 | 0.4510 | 0.5444 | 0.4226 |

3) SVM3 tapping ring finger (2) VS flick (3)

**feat_matrix2_3 =**

| | | | | | | | |
|---|---|---|---|---|---|---|---|
| 0.2024 | 0.2978 | 0.1122 | 0.4444 | 1.0000 | 0.9744 | 0.9515 | 0.9310 |
| 0.1376 | 0.2769 | 0.0986 | 0.3125 | 1.0000 | 1.0000 | 1.0000 | 1.0000 |
| 0.2169 | 0.3175 | 0.1317 | 0.3772 | 1.0000 | 0.8644 | 0.8960 | 0.8750 |
| 0.1686 | 0.2999 | 0.1282 | 0.4348 | 1.0000 | 0.9150 | 0.9529 | 0.9186 |
| 1.0000 | 0.9186 | 1.0000 | 0.8523 | 0.1350 | 0.3248 | 0.2516 | 0.2532 |
| 1.0000 | 0.9637 | 0.7673 | 1.0000 | 0.2838 | 0.5257 | 0.4421 | 0.4905 |
| 1.0000 | 1.0000 | 0.8948 | 0.9539 | 0.3780 | 0.6468 | 0.5995 | 0.6065 |
| 1.0000 | 0.7280 | 0.9629 | 0.7493 | 0.2654 | 0.5918 | 0.6290 | 0.3385 |

As already described, it is possible to distinguish four sub-matrixes in terms of magnitude of difference. Values in the matrix have been normalized two times. First each row and then each column i.e. each features (as required by SVM). In summary, the training phase of each SVM requires two templates related to two different gestures for every sensor and four repetitions of each gesture for every microphone.

### RESULTS FOR 10 SUBJECTS

Concerning 3 gestures recognition, 10 (5 F, 5M) different subjects have been analyzed on 15 repetitions of each gesture for the validation process. Following table shows the results and some observations:

| | Errors | accuracy | Note |
|---|---|---|---|
| S1 (F) | 0 | 100% | |
| S2 (M) | 0 | 100% | |
| S3 (M) | 3 | 93.3% | Lower threshold |
| S4 (M) | 2 | 95.5% | Very low threshold |
| S5 (M) | 1 | 97.7% | |
| S6 (F) | 0 | 100% | |
| S7 (F) | 0 | 100% | |
| S8 (F) | 0 | 100% | Lower threshold |
| S9 (M) | 0 | 100% | |
| S10 (F) | 0 | 100% | Lower threshold |

## Claims

1. A wrist-worn sensor for measuring wrist tendon forces corresponding to specific finger motions comprising:
a. an array of cantilever piezoelectric sensors wherein the piezoelectric sensors emit electric currents generated upon pressure from wrist tendons on the tip of the piezoelectric sensors;
b. a processing module configured for converting the electric currents generated upon pressure from wrist tendons into signals and for processing the signals for identification of one or more specific finger motions;
c. a flexible PCB connecting the array of cantilever piezoelectric sensors to the processing module.

2. The wrist-worn sensor of Claim 1 wherein the array of piezoelectric sensors is configured to have a spatial resolution of less than 2 mm.

3. The wrist-worn sensor of Claim 1 wherein the cantilever sensors are configured in a linear array, optionally wherein the linear array comprises four piezo- electric sensors with partially overlapping sensor areas.

4. The wrist worn sensor of Claim 3 where the array of cantilever piezoelectric sensors is positioned proximally to any or all of a wearer's Flexor Carpi Ulnaris Tendon, Flexor Digitorum Profundus Tendon and Flexor Digitorum Superficialis Tendon.

5. The wrist worn sensor of Claim 3 where the array of cantilever piezoelectric sensors is configured to substantially capture the tension applied to each tendon in the wrist.

6. The wrist-worn sensor of Claim 1 wherein the sensors are positioned at an angle greater than 10 degrees relative to the flexible PCB.

7. The wrist-worn sensor of Claim 1 wherein the piezoelectric sensors are embedded in an elastomeric material, optionally wherein the elastomeric material is selected from the list consisting of silicone rubber, polymer foam and polymer elastomer, and/or wherein the elastomeric material filters out low amplitude high frequency signals.

8. A computer interface, comprising the wrist-worn sensor of Claim 1 and a controller module configured to cause one or more computing devices to automatically execute one or more specific commands upon identification of one or more of the specific finger motions, optionally wherein the computer interface communicates wirelessly with one or more computing devices and/or further comprising a button placed in contact with a user's wrist so as to be triggered by the user flexing the wrist and causing the activation of the device from a sleeping, power-saving mode to an active acquisition mode.

9. A process for detecting specific finger movements based on wrist-tendon forces, the process comprising the steps of:
a. sensing one or more electric signals produced by an array of cantilever piezoelectric sensors generated upon pressure of wrist tendons applied to the tip of the sensors;
b. extracting a set of characteristic features from the electric signal produced by the array of cantilever piezoelectric sensors;
c. feeding the characteristic features to a trained classifier;
d. identifying one or more specific finger gestures associated with specific classes of the trained classifier; and
e. automatically directing one or more computing devices to execute one or more commands corresponding to one or more of the identified finger gestures.

10. The process of claim 9 further comprising the step of performing an initial calibration of the sensors which evaluates gesture generated signals associated with a subset of user finger gestures to determine expected signals during the finger-gesture identification step.

11. The process of Claim 9 further comprising the step of calibrating the controller by automatically identifying the parameters needed to run a software program installed in the module or in one or more external computing devices, the software program receiving the signals and identifying the parameter for the training following a protocol of specific finger gestures.

12. The process of claim 9, wherein the feature extraction step further comprises the steps of considering all electric signals coming from the sensors during each finger movement and gesture, band-pass filtering said signals to limit the data to a predetermined amount, and analyzing the signals by means of a feature extractor.

13. The process of claim 9, wherein the feature extraction step analyzes the signals in order to obtain a set of features describing the signals to be compared with other signal features coming from other finger movements and gestures.

14. The process of claim 9, wherein the features are selected from the list consisting of time domain features and frequency domain features.

15. The process of Claim 9 further comprising a step of disabling one or more of the sensors during rest.
